# EUROPEAN PATENT APPLICATION

(11) **EP 4 512 886 A1**
(43) Date of publication of application: **26.02.2025**
(21) Application number: 23933309.9
(22) Date of filing: 17.04.2023
(51) Int. Cl.: C12M 1/34

(54) **NUCLEIC ACID TESTING DEVICE AND NUCLEIC ACID TESTING METHOD**

(71) Applicant: Chang Gung University, Taoyuan County 33302, Taiwan (TW)
(72) Inventor: WU, Min-Hsien, Taoyuan City, 33302 Taiwan (TW); HSIEH, Ping-Han, Taoyuan City, 33302 Taiwan (TW); CHU, Po-Yu, Taoyuan City, 33302 Taiwan (TW)
(74) Representative: Zaboliene, Reda
(86) International application number: PCT/CN2023/088637
(87) International publication number: WO 2024/216427

(57) **Abstract**

This invention provides a nucleic acid detection device and a nucleic acid detection method. The nucleic acid detection device comprises a bearing body, a detection body, and a motion body. The bearing body comprises a substrate part and a bridge-shaped part, the bridge-shaped part is arranged on the upper surface of the substrate part, and a sample pad is arranged on the bridge-shaped part. A channel is formed between the bridge-shaped part and the substrate part, the motion body is detachably arranged in the channel, and the motion body comprises at least one liquid absorption area and non-liquid absorption area. The nucleic acid detection method is a method for nucleic acid amplification using the sample pad and nucleic acid detection using the nucleic acid detection device. This invention solves the problem that currently lacking a device for use at home and having nucleic acid amplification and detection functions.

## Description

### REFERENCE TO RELATED APPLICATIONS

The present invention is based on, and claims priority from, international application number PCT/CN2023/088637, filed on April 17, 2023, the disclosure of which is hereby incorporated by reference herein in its entirety.

### REFERENCE TO RELATED APPLICATIONS

The present invention contains one or more sequences in a computer readable format in an accompanying file titled "226073.xml", which is 3.76 KB in size and was created March 29, 2024, the contents of which are incorporated herein by reference in their entireties.

### FIELD OF THE INVENTION

The present invention relates to the nucleic acid detection field, and in particular, it relates to a nucleic acid detection device and a nucleic acid detection method.

### BACKGROUND OF THE INVENTION

Nucleic acid amplification and nucleic acid detection have been widely applied to the fields of in-vitro diagnosis, life science research, and the like at present, and with the development of science and technology, devices for nucleic acid amplification and nucleic acid detection have gradually developed towards miniaturization, low cost, and convenience. However, the existing devices for nucleic acid amplification generally need to strictly control the temperature during nucleic acid amplification, and therefore said nucleic acid amplification needs to be provided with temperature control devices, which leads to difficulty in reduction of the volume of the said nucleic acid devices, which makes the volume and the cost are difficult to reduce, and a further nucleic acid detection is required after completing nucleic acid amplification to confirm the results of nucleic acid amplification. Depending on the aforementioned reasons, most of the devices available on the market have only nucleic detection functions such as virus rapid test kits, but these types of rapid test kits may be prone to false negatives because the initial viral load is not high enough, so lacking a nucleic acid detection device that can be used at home and has nucleic acid amplification function and nucleic acid detection function on the market is a problem to be solved.

### SUMMARY OF THE INVENTION

An object of the present invention is to solve the problem of the lack of a nucleic acid detection device that can be used at home and that has a nucleic acid amplification function and nucleic acid detection function at present.

Based on the object of the present invention, the present invention provides a nucleic acid detection device, comprising a bearing body, a detection body, and a motion body, wherein the bearing body comprises a substrate part and bridge-shaped part; wherein the bridge-shaped part is disposed on the upper surface of the substrate part, and a channel is formed between the bridge-shaped part and the substrate part; wherein the detection body comprises a starting pad, at least one control line, and at least one test line; wherein the detection body is disposed on the upper surface of the substrate part, and the starting pad of the detection body is located in the channel; wherein the bridge-shaped part is provided with a window for viewing a detection result shown by the at least one control line and the at least one test line of the detection body; wherein a sample pad is provided at a position, corresponding to the starting pad of the detection body, of the bridge-shaped part; wherein the motion body is detachably provided in the channel, and the motion body comprises at least one liquid absorption area and at least one non-liquid absorption area.

In an embodiment of the present invention, the sample pad comprises a nucleic acid adsorption layer.

In an embodiment of the present invention, the sample pad further comprises a nucleic acid extraction layer, and the nucleic acid extraction layer is disposed on the upper surface of the nucleic acid adsorption layer.

In an embodiment of the present invention, the sample pad further comprises a nucleic acid amplification reagent.

In an embodiment of the present invention, the nucleic acid detection device further comprises a guide body, one end of the guide body is connected to the upper surface of the motion body, and the guide body covers the upper surface of the sample pad of the bridge-shaped part of the bearing body; wherein a plurality of openings is provided on the guide body, wherein at least one of the plurality of openings corresponds to the liquid absorption area, and at least one of the plurality of openings corresponds to the non-liquid absorption area.

In an embodiment of the present invention, at least one of the openings of the plurality of the openings corresponding to the liquid absorption area of the motion body is further provided with a nucleic acid extraction layer.

The present invention provides a nucleic acid detection method, comprising the following steps in sequence: a nucleic acid detection device providing step: providing the nucleic acid detection device as claimed in claim 1; a sample adding step: after the liquid absorption area of the motion body corresponds to the sample pad, adding a sample to the tested to the sample pad, wherein the sample to be tested comprises a target nucleic acid; a nucleic acid amplification step: after the non-liquid absorption area of the motion body corresponds to the sample pad, enabling the target nucleic acid in the sample pad to react with a nucleic acid amplification reagent to form amplicons of the target nucleic acid; a nucleic acid detection step: after the motion body is pulled away from the channel, pressing the bridge-shaped part to make the sample pad on the bridge-shaped part to contact with the starting pad of the detection body, and then adding an eluent to the sample pad, so that the amplicons of the target nucleic acid in the sample pad flow to the starting pad of the detection body along with the eluent, and then flow to the at least one control line and the at least one test line of the detection body from the starting pad, wherein the amplicons of the target nucleic acid is captured on the at least one test line.

In an embodiment of the present invention, after completing the sample adding step, further comprises a first cleaning step, wherein the first cleaning step is to add a first cleaning liquid to the sample pad after the liquid absorption area of the motion body corresponds to the sample pad.

In an embodiment of the present invention, after completing the nucleic acid amplification step, further comprises a second cleaning step, wherein the second cleaning step is to add a second cleaning liquid to the sample pad after the liquid absorption area of the motion body corresponds to the sample pad.

In an embodiment of the present invention, after completing the sample adding step, further comprises a first cleaning step, and after completing the nucleic acid amplification step, further comprises a second cleaning step; wherein the first cleaning step is to add a first cleaning liquid to the sample pad after the liquid absorption area of the motion body corresponds to the sample pad; wherein the second cleaning step is to add a second cleaning liquid to the sample pad after the liquid absorption area of the motion body corresponds to the sample pad.

In summary, the nucleic acid detection device and the nucleic acid detection method provided by the present invention can let a user conveniently accomplish a whole process from adding the sample to be tested and nucleic acid amplification to nucleic acid detection in the home environment, and solve the problem of the lack of the existing nucleic acid detection device that can be used at home and has the functions of nucleic acid amplification and nucleic acid detection.

### BRIEF DESCRIPTION OF THE DRAWINGS

FIG. 1 is a three-dimensional schematic diagram of a nucleic acid detection device in an embodiment.
FIG. 2 is a schematic top view of FIG. 1.
FIG. 3 is a schematic diagram of removing a guide body in FIG. 2.
FIG. 4 is a schematic diagram of removing a bridge-shaped part and a motion body in FIG. 3.
FIG. 5 is a three-dimensional schematic diagram of a bearing body and a detection body in FIG. 1.
FIG. 6 is a cross-sectional view taken along line A-A in FIG. 2.
FIG. 7 is a schematic side view of a first form of the bearing body.
FIG. 8 is a schematic side view of a second form of the bearing body.
FIG. 9 is a schematic side view of a third form of the bearing body.
FIG. 10 is a schematic side view of a fourth form of the bearing body.
FIG. 11 is a step diagram of a nucleic acid detection method of the present invention.

### EXPLANATION OF REFERENCE SIGNS

1: nucleic acid detection device
2: bearing body
3: detection body
4: motion body
5: guide body
6: guide mark
20: substrate part
22: bridge-shaped part
30: starting pad
32: binding pad
34: reaction film
36: absorption pad
40: motion substrate
50: first pattern mark
51: second pattern mark
52: third pattern mark
54: first text mark
55: second text mark
56: third text mark
57: fourth text mark
58: fifth text mark
59: sixth text mark
220: sample pad
222: channel
224: window
340: control line
342: test line
2200: nucleic acid extraction layer
2202: nucleic acid adsorption layer
22a: first sidewall
22b: connecting face
22c: second sidewall
A1: first liquid absorption area
A2: second liquid absorption area
A-A: section line
AP1: first liquid absorption pad
AP2: second liquid absorption pad
B: non-liquid absorption area
Bu1: first engaging part
Bu2: second engaging part
O1: first opening
O2: second opening
O3: third opening
S10: nucleic acid detection device providing step
S20: sample adding step
S25: first cleaning step
S30: nucleic acid amplification step
S35: second cleaning step
S40: nucleic acid detection step
WP: waterproof pad

### DETAILED DESCRIPTION OF THE PREFERRED EMBODIMENTS

"An embodiment" described in the whole description means that a specific feature, structure, or characteristic is included in at least one embodiment. Therefore, "an embodiment" set forth in the description does not necessarily refer to the same embodiment. In addition, a specific feature, structure, or characteristic may be implemented in any manner in one or more embodiments.

The "first", "second", "third", "fourth", and "fifth" mentioned in the whole description are only for descriptive purposes and should not be understood as indicating or implying relative importance.

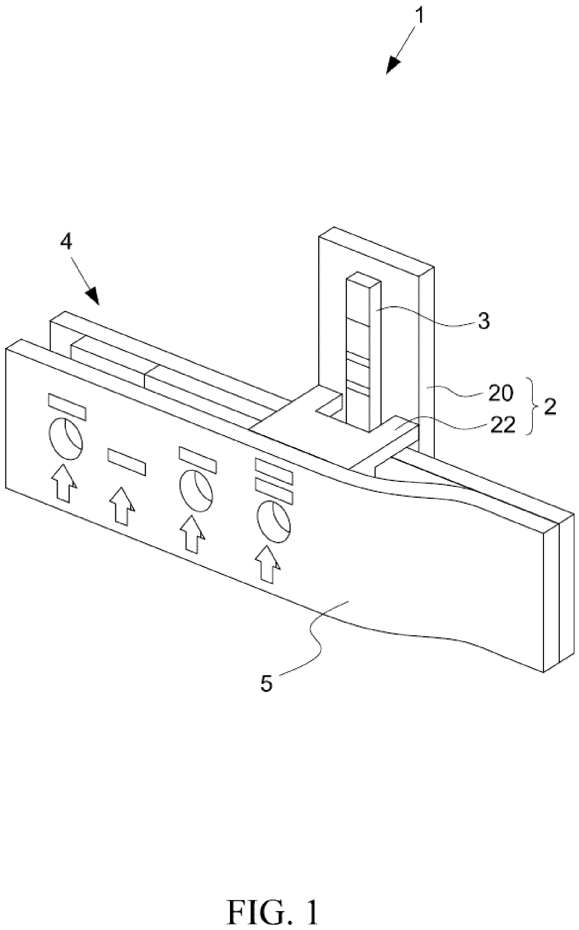

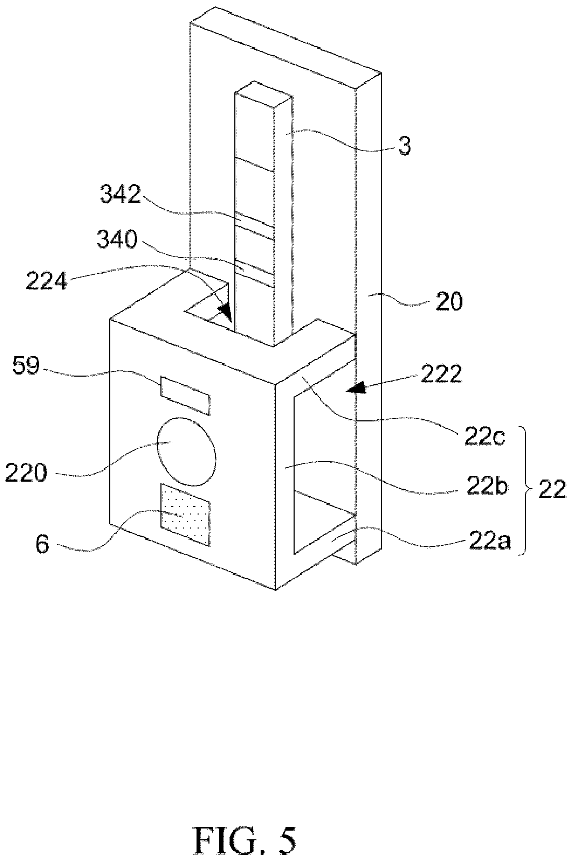

Referring to FIG. 1, FIG. 4, FIG. 5, FIG. 6, FIG. 7, FIG. 8, FIG. 9, and FIG. 10, the present invention provides a nucleic acid detection device 1, comprising a bearing body 2, a detection body 3, and a motion body 4. The bearing body 2 comprises a substrate part 20 and a bridge-shaped part 22. The bridge-shaped part 22 is disposed on the upper surface of the substrate part 20 of the bearing body 2, and a channel 222 is formed between the bridge-shaped part 22 and the substrate part 20. The detection body 3 comprises a starting pad 30, at least one control line 340, and at least one test line 342. The detection body 3 is disposed on the upper surface of the substrate part 20, and the starting pad 30 of the detection body 3 is located in the channel 222. One end of the bridge-shaped part 22 is provided with a window 224, and the window 224 is used for allowing the detection body 3 to pass through the end of the bridge-shaped part 22. A sample pad 220 is provided at a position, corresponding to the starting pad 30 of the detection body 3, of the bridge-shaped part 22. The motion body 4 is detachably provided in the channel 222, and the motion body 4 comprises at least one liquid absorption area and at least one non-liquid absorption area B.

Referring to FIG. 1, FIG. 2, FIG. 3, FIG. 5, FIG. 6, and FIG. 7, in an embodiment of the present invention, the substrate part 20 of the bearing body 2 is used for bearing the bridge-shaped part 22, detection body 3, and motion body 4. The bridge-shaped part 22 includes a first sidewall 22a, a connecting face 22b, and a second sidewall 22c, one side of the first sidewall 22a is connected to the upper surface of the substrate part 20 and is at a position close to the starting pad 30, and the other side of the first sidewall 22a is connected to one side of the connecting face 22b, the other side of the connecting face 22b is connected to one side of the second sidewall 22c, the other side of the second sidewall 22c is connected to the upper surface of the substrate part 20 and is at a position close to the at least one control line 340 and at least one test line 342, the connection between the second sidewall 22c and the substrate part 20 is provided with the window 224, the window 224 is used for allowing the detection body 3 to pass through the second sidewall 22c. A sample pad 220 is provided at a position, corresponding to the starting pad 30 of the detection body 3, of the connecting face 22b.

Referring to FIG. 1, FIG. 2, FIG. 3, and FIG. 4, in an embodiment of the present invention, the detection body 3 is a lateral flow immunochromatography paper, and the detection body 3 is fixed on the upper surface of the substrate part 20 of the bearing body 2 in an adhesive manner. The detection body 3 includes the starting pad 30, a binding pad 32, a reaction film 34, and a absorption pad 36, the starting pad 30 is provided on one end of the detection body 3, the absorption pad 36 is provided on the other end of the detection body 3, one end of the starting pad 30 is connected to one end of the binding pad 32, the other end of the binding pad 32 is connected to one end of the reaction film 34, the other end of the reaction film is connected to one end of the absorption pad 36. The reaction film 34 includes the at least one control line 340 and the at least one test line 342.

Referring to FIG. 1, FIG. 2, FIG. 3, FIG. 4, FIG. 5, FIG. 6, and FIG. 7, in an embodiment of the present invention, the detection body 3 passes through the second sidewall 22c through the window 224, thus the at least one control line 340 and the at least one test line 342 of the detection body 3 are located outside the bridge-shaped part 22, so that the user can see the at least one control line 340 and the at least one test line 342 of the detection body 3. Therefore, the main function of the window 224 on the bridge-shaped part 22 is to allow the user to see the at least one control line 340 and the at least one test line 342, thereby the user can conveniently judge the detection result.

The bearing body 2 is not limited to the form of the bearing body 2 as mentioned above, and the form of the bearing body 2 as mentioned above is hereinafter referred to as the first form of the bearing body 2. Other forms of the bearing body 2 are exemplified, representing the second form, the third form, and the fourth form of the bearing body 2, as embodiments, respectively. The difference between the second form, the third form, and the fourth form of the bearing body 2 and the first form of the bearing body 2 is that the structure of the bridge-shaped part 22 and the position of the window 224 on the bridge-shaped part 22, and each form of the bearing body 2 is described respectively below with reference to FIG. 1 to FIG. 10.

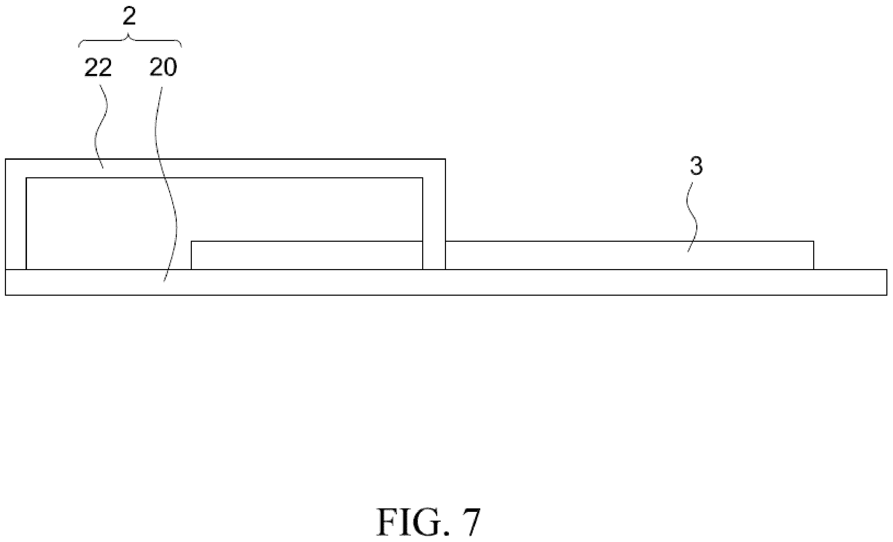

Referring to FIG. 7, FIG. 7 is a schematic side view of the first form of the bearing body 2, detailed may refer to the paragraphs described above, and further refer to FIG. 1 to FIG. 6.

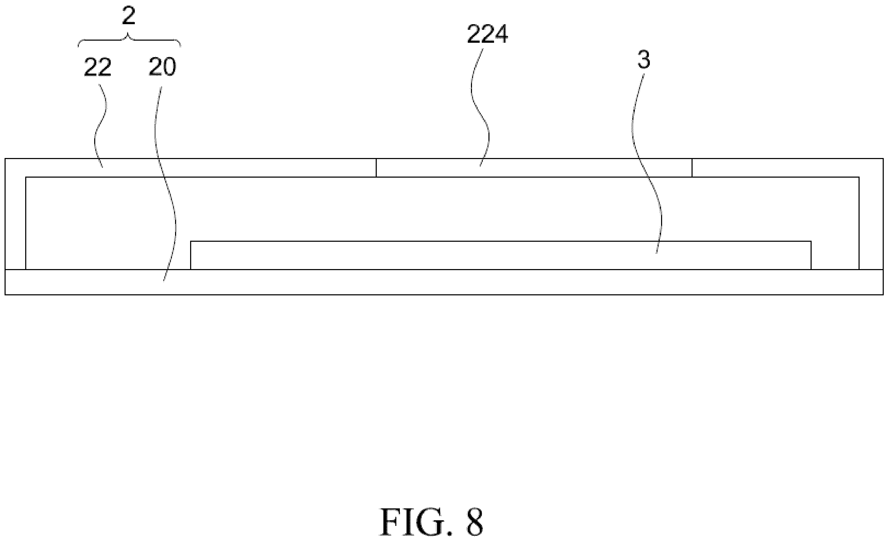

Referring to FIG. 8, FIG. 8 is a schematic side view of the second form of the bearing body 2, one end and the other end of the bridge-shaped part 22 are disposed on the upper surface of the substrate part 20, the difference between the second form of the bearing body 2 and the first form of the bearing body 2 is that the bridge-shaped part 22 covers the entire detection body 3, and the bridge-shaped part 22 is provided with the sample pad 220 corresponding to the starting pad 30 of the detection body 3, and the bridge-shaped part 22 is provided with the window 224 at a position corresponding to the at least one control line 340 and the at least one test line 342 of the detection body 3.

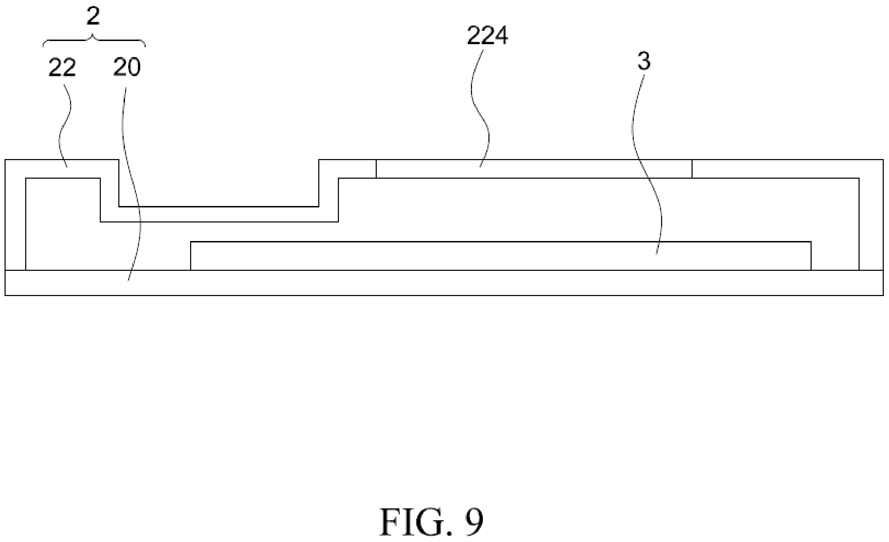

Referring to FIG. 9, FIG. 9 is a schematic side view of the third form of the bearing body 2, the third form of the bearing body 2 is basically consistent with the second form of the bearing body 2, with the difference that the sample pad 220 is disposed on the bridge-shaped part 22 to present in form of the groove, thereby shortening the distance between the sample pad 220 and the starting pad 30 of the detection body 3.

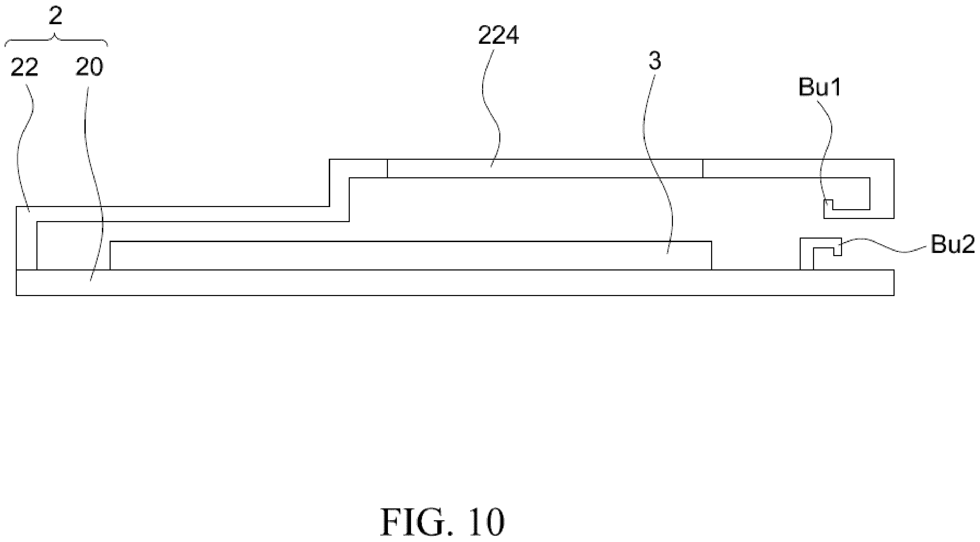

Referring to FIG. 10, FIG. 10 is a schematic side view of the fourth form of the bearing body 2, one end of the bridge-shaped part 22 is disposed on the upper surface of the substrate part 20, and the bridge-shaped part 22 is stepped, thereby shortening the distance between the sample pad 220 and the starting pad 30 of the detection body 3, in addition, the other end of the bridge-shaped part 22 is provided with a first engaging part Bu1, and one end of the substrate part 20 is provided with a second engaging part Bu2, wherein the first engaging part Bu1 is configured to be engagingd with the second engaging part Bu2, and when the first engaging part Bu1 and the second engaging part Bu2 are engagingd, the bridge-shaped part 22 is close to the sample pad 220, so that the sample pad 220 is in contact with the starting pad 30 of the detection body 3.

In the aforementioned embodiments of the bearing body 2, the window 224 of the first form of the bearing body 2 is an opening. In the second form, the third form, and the fourth form of the bearing body 2, the form of the window 224 may be an opening or a light-transmitting material, wherein the light-transmitting material is, for example, a transparent plastic, preservative film, or a transparent glass plate, but the practice is not limited thereto and may be other materials with light transmittance, so that the user can view the detection result shown by the at least one control line 340 and at least one test line on the detection body 3 from the outside of bridge-shaped part 22.

Referring to FIG. 1, FIG. 2, FIG. 3, FIG. 5, and FIG. 6, in an embodiment of the present invention, the motion body 4 includes a motion substrate 40, and the motion substrate 40 includes the at least one liquid absorption area and the at least one non-liquid absorption area B, wherein the liquid absorption area refers to the area where the liquid absorption pad is disposed on the motion body 4, the non-liquid absorption area B refers to area where the waterproof pad is disposed on the motion body 4, and both the liquid absorption pad and waterproof pad WP are all disposed on the upper surface of the motion substrate 40 in an adhesive manner, the motion body 4 can move along the direction of the channel 222, so that the liquid absorption area of the motion body 4 corresponds to the sample pad 220, or the non-liquid absorption area B of the motion body 4 corresponds to the sample pad 220, that is, through the movement of the motion body 4, the sample pad 220 is stacked on the upper surface of the liquid absorption pad or the waterproof pad WP, and in addition, the motion body 4 may also be pulled away from the channel 222. The aforementioned context is for example only, the practice is not limited thereto, the non-liquid absorption area B of the motion body 4 may also refer to the area where no water absorption pad is provided, so that when the non-liquid absorption area B corresponds to sample pad 220 because the sample pad 220 is not in contact with the water absorption pad, the liquid added to the sample pad 220 will remain in the sample pad 220. In a manner of moving the motion body 4, a part of the motion body 4 located outside the channel 222 can be pinched by hand or a clamping tool, and the motion body 4 is pushed or pulled to move the motion body 4 along the direction of the channel 222, wherein the clamping tool is, for example, a tool such as tweezers and a binder clip, which is not particularly limited in the present invention.

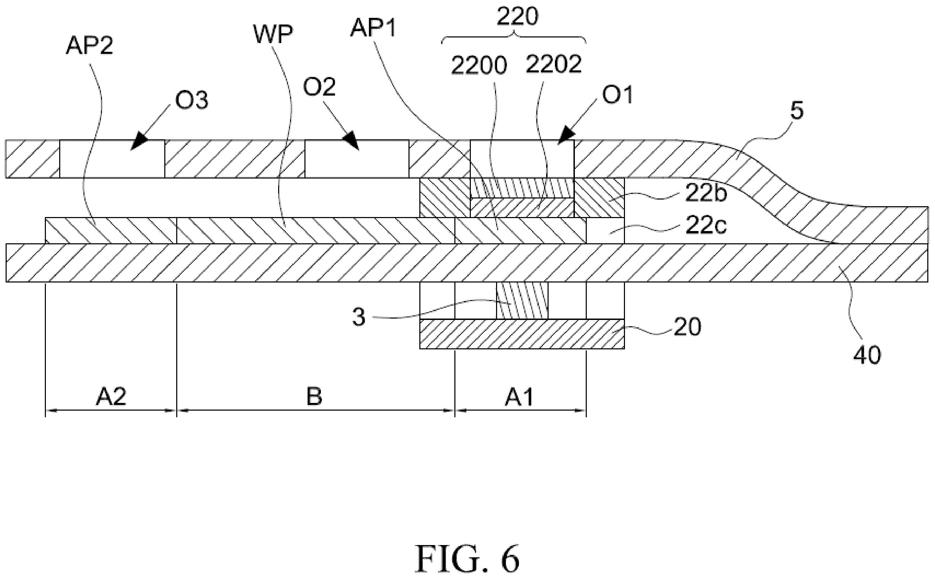

Referring to FIG. 6, in an embodiment of the present invention, the sample pad 220 includes a nucleic acid extraction layer 2200 and a nucleic acid adsorption layer 2202, the nucleic acid extraction layer 2200 is disposed on the upper surface of the nucleic acid adsorption layer 2202; in another embodiment of the present invention, the sample pad 220 includes only the nucleic acid adsorption layer 2202.

Referring to FIG. 1, FIG. 2, FIG. 5, and FIG. 6, in an embodiment of the present invention, the nucleic acid detection device 1 further includes a guide body 5, one end of the guide body 5 is connected to the upper surface of the motion body 4, and when the motion body 4 is disposed in the channel 222, the guide body 5 covers the upper surface of the sample pad 220, the guide body 5 is provided with a plurality of openings for exposing the upper surface of the sample pad 220, wherein at least one of the plurality of openings corresponds to the liquid absorption area of the motion body 4, and at least one of the plurality of openings corresponds to the non-liquid absorption area B of the motion body 4.

Referring to FIG. 1, FIG. 2, and FIG. 6, in an embodiment of the present invention, the nucleic acid extraction layer 2200 is further provided in at least one opening in the plurality of the openings of the guide body 5 corresponding to the liquid absorption area of the motion body 4, and the other openings of the plurality of openings are used for exposing the upper surface of the sample pad 220.

Referring to FIG. 1, FIG. 2, FIG. 3, FIG. 5, and FIG. 6, in an embodiment of the present invention, a plurality of cue marks is further included, and the plurality of cue marks is marked on the guide body 5 and the bearing body 2, wherein the cue marks are marks for hinting the sequence of the nucleic acid detection method of the present invention, and may be a text mark, a pattern mark, or a combination thereof, and the marking manner of each cue mark may be a marking manner such as printing, imprinting, punching, sticker marking, and marking by using a fluorescent marker, which is not particularly limited in the present invention. When the plurality of cue markers is marked in a punching manner, the connecting face 22 of the bridge-shaped part22 can further include a guide mark 6, the guide mark 6 is used for matching with a plurality of the cue marks, thereby the position of the motion body 4 corresponding to the sample pad 220 at present is prompted to the user, wherein the guide mark 6 may be a text mark, a pattern mark, or a combination thereof, and the marking manner of each guide mark 6 may be a marking manner such as printing, imprinting, punching, sticker marking, and marking by using a fluorescent marker, so that when the cue mark is not overlapped with the guide mark 6, the guide mark will be covered below the guide body 5, and if the cue mark is overlapped with guide mark 6, the cue mark will expose the guide mark 6.

Referring to FIG. 1 to FIG. 11, the present invention provides a nucleic acid detection method, which sequentially includes a nucleic acid detection device providing step S10, a sample adding step S20, a nucleic acid amplification step S30, and a nucleic acid detection step S40; wherein the nucleic acid detection device providing step S10 is to provide the nucleic acid detection 1 of the present invention; wherein the sample adding step S20 is to add the sample to be tested to the sample pad 220 after the liquid absorption area of the motion body 4 corresponds to the sample pad 220, wherein the sample to be tested includes a target nucleic acid; wherein the nucleic acid amplification step S30 is to make the target nucleic acid in the sample pad 220 to react with a nucleic acid amplification reagent to form amplicons of the target nucleic acid after the non-liquid absorption area B of the motion body 4 corresponds to the sample pad 220; wherein the nucleic acid detection step S40 is to pull the motion body 4 away from the channel 222, then press the bridge-shaped part 22 to make the sample pad 220 in contact with the starting pad 30, and then add the eluent to the sample pad 220, so that the amplicons of the target nucleic acid in the sample pad 220 flow to the starting pad 30 of the detection body 3 along with the eluent, then flow to the at least one control line 340 and the at least one test line 342 of the detection body 3 from the starting pad 30, wherein the amplicons of the target nucleic acid is captured at the at least one test line 342.

Referring to FIG. 1 to FIG. 11, in an embodiment of the present invention, after completing the sample adding step S20, further comprises a first cleaning step S25, the first cleaning step S25 is to add a first cleaning liquid to the sample pad 220 after the liquid absorption area of the motion body 4 corresponds to the sample pad 220.

Referring to FIG. 1 to FIG. 11, in an embodiment of the present invention, after completing the nucleic acid amplification step S30, further comprises a second cleaning step S35, the second cleaning step S35 is to add a second cleaning liquid to the sample pad 220 after the liquid absorption area of the motion body 4 corresponds to the sample pad 220.

Referring to FIG. 1 to FIG. 11, in an embodiment of the present invention, after completing the sample adding step S20, further comprises a first cleaning step S25, and after completing the nucleic acid amplification step S30, further comprises a second cleaning step S35; wherein the first cleaning step S25 is to add a first cleaning liquid to the sample pad 220 after the liquid absorption area of the motion body 4 corresponds to the sample pad 220; wherein the second cleaning step S35 is to add a second cleaning liquid to the sample pad 220 after the liquid absorption area of the motion body 4 corresponds to the sample pad 220.

Referring to FIG. 1 to FIG. 11, in an embodiment of the present invention, the "to make the target nucleic acid in the sample pad 220 to react with a nucleic acid amplification reagent" in the nucleic acid amplification step S30 is to add the nucleic acid amplification reagent to the sample pad 220, so that the nucleic acid amplification reagent reacts with the target nucleic acid in the sample pad 220 to form the amplicons of the target nucleic acid; in another embodiment of the present invention, the " to make the target nucleic acid in the sample pad 220 to react with a nucleic acid amplification reagent" in the nucleic acid amplification step S30 is that the sample pad 220 further includes the nucleic acid amplification reagent, wherein the nucleic acid amplification reagent maybe in a freeze-drying form in the sample pad 220, so that after completing the sample adding step S20, then making the non-liquid absorption area B of the moving the motion body 4 to correspond to the sample pad 220, and then the target nucleic acid in the sample pad 220 reacts with the nucleic acid amplification reagent to form the amplicons of the target nucleic acid.

Various embodiments are provided below and are specifically described with reference to drawings, and the detailed content in each embodiment is as described in the following paragraphs, wherein each embodiment includes Embodiment 1, Embodiment 2, and Embodiment 3.

In the following embodiments, the sample to be tested uses *Agrobacterium tumefaciens* suspension or genomic nucleic acid of *Agrobacterium tumefaciens* as an example, and the following *Agrobacterium tumefaciens* is abbreviated as "Agrobacterium". However, the practice of the sample to be tested is not limited thereto, and the sample to be tested may be viruses or organisms such as microorganisms, tissues, cells, bacteria, fungi, algae, protozoa, and fungus-like protists. In addition, the sample to be tested may also be a cell-free nucleic acid, wherein the cell-free nucleic acid may be DNA or RNA, and the present invention is not particularly limited, wherein the cell-free nucleic acid may be a nucleic acid obtained from nucleic acid extraction of viruses or organisms such as microorganisms, tissues, cells, bacteria, fungi, algae, protozoa, and fungus-like protists in advance, wherein nucleic acid extraction can be, for example, releasing nucleic acid from the viruses or the aforementioned biological using a lysis solution or adding the viruses or the aforementioned biological to a film containing a lysis solution that is dried to release the nucleic acid, wherein the film is, for example, glass fiber membrane, filter paper, a gel, non-woven fabric, cellulose membrane, polyester membrane, or polypropylene film, and the present invention is not particularly limited.

In each of the following embodiments, the target nucleic acid uses the *virG* gene of Agrobacterium as an example, wherein a part of the nucleotide sequence of the *virG* gene of Agrobacterium is shown as SEQ ID NO: 1, and referring to Table 1 below, Table 1 shows sequence name and corresponding nucleotide sequence, but the practice is not particularly limited thereto, and the target nucleic acid to be detected can be selected according to practical requirements, the present invention is not limited thereto, wherein the target nucleic acid can be DNA or RNA.

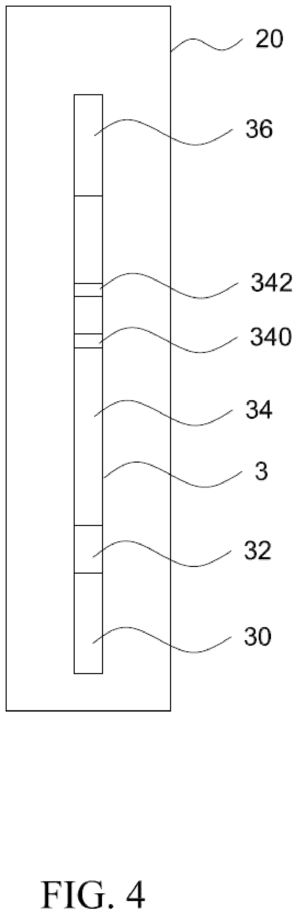

Referring to FIG. 4 and FIG. 6, in the following embodiments, the nucleic acid amplification all adopts a recombinase polymerase amplification technology, thus the nucleic acid amplification reagent is a recombinase polymerase amplification (RPA) reagent, wherein the recombinase polymerase amplification reagent includes a recombinase, a single-stranded DNA-binding protein (SSB), strand-displacing polymerase, a forward primer, and a reverse primer. The forward primer and the reverse primer are used for binding to SEQ ID NO: 1 as an origin of nucleic acid amplification, wherein the nucleotide sequence of the forward primer as shown in SEQ ID NO: 2, and refer to Table 1 below, Table 1 shows sequence name and corresponding nucleotide sequence, the 5' end of the nucleotide sequence of the forward primer is binding with a chemical marker, wherein the chemical marker is biotin. The nucleotide sequence of the reverse primer as shown in SEQ ID NO: 3, and refer to Table 1 below, Table 1 shows sequence name and corresponding nucleotide sequence, and the 5' end of the nucleotide sequence of the reverse primer is binding with a fluorescent marker, wherein the fluorescent marker is fluorescein isothiocyanate (FITC), thus after the nucleic acid amplification is subsequently performed on the target nucleic acid, the forward primer and the reverse primer are left on the amplicons of the target nucleic acid, that is, the amplicons of the target nucleic acid is provided with the chemical marker and the fluorescent marker so as to facilitate subsequent detection of the amplicons of the target nucleic acid by using the detection body 3, wherein due to the amplicons of the target nucleic acid is provided with the fluorescent marker, thus a judgment can also be made based on whether a fluorescence signal is generated on the test line 342 of the detection body 3 or not, and if the fluorescence signal is generated, indicating that the amplicons of the target nucleic acid is detected, but the practice is not limited thereto, the fluorescent marker can also be other fluorescent substances commonly used for primer labeling, the chemical marker can also be other chemical base modifications commonly used for primer labeling, and the forward primer and the reverse primer can be designed according to the target substance to be detected, and in addition, in practice, the forward primer can also be labeled with the fluorescent marker , and the reverse can also be labeled with the chemical marker, which is not particularly limited in the present invention; wherein the recombinase binds to the forward primer or the reverse primer, identifies a homologous DNA on the target nucleic acid in the sample to be tested, and simultaneously guides the forward primer or the reverse primer to form a D-loop and single-strand DNA interval, and the single-stranded DNA-binding protein binds to single-strand DNA interval to stabilize the structure, the strand-displacing polymerase is then perform the nucleic acid amplification by taking the position where the forward primer or the reverse primer is bound as an origin, and then the amplicons of the target nucleic acid is continuously generated by repeating the aforementioned nucleic acid amplification, but the practice is not limited thereto, the nucleic acid amplification can also be an isothermal nucleic acid amplification such as Rolling Circle Amplification (RCA) technology, Helicase-Dependent Amplification (HDA) technology, Strand Displacement Amplification (SDA) technology, Nicking Enzyme Amplification Reaction (NEAR) technology, Nucleic acid sequence-Based amplification (NASBA) technology, exponential amplification reaction (EXPAR) technology, and Loop mediated isothermal amplification (LAMP) technology, and use the corresponding isothermal nucleic acid amplification reagent according to the isothermal amplification technology used, that is, the reagent such as Rolling Circle Amplification reagent, Helicase-Dependent Amplification reagent, Strand Displacement Amplification reagent, Nicking Enzyme Amplification Reaction reagent, Nucleic acid sequence-Based amplification reagent, exponential amplification reaction reagent, and Loop mediated isothermal amplification reagent, and meanwhile, if the sample to be tested is RNA, or the sample to be tested is lysed by nucleic acid extraction layer 2200 to release RNA, then a nucleic acid reverse transcription reagent can be further added in the nucleic acid amplification.

**Table 1: nucleotide sequence corresponding to SEQ ID NO: 1, SEQ ID NO: 2, and SEQ ID NO: 3, and the direction of the nucleotide sequence from left to right is from the 5 'end to the 3 'end**

| Sequence Name | Nucleotide Sequence (5' end to 3' end) |
|---|---|
| SEQ ID NO: 1 | ttgaggagacggataaagttgttgcactcgagctaggagcaagtgattttatcgctaagccgtttagtacgagagagtttcttgcacgcatt cgggttgccttgcgcgtgcgccccaacgttgtccgctccaaagaccgacggtctttttgttttactgactggacacttaatctcaggcaac gtcgc |
| SEQ ID NO: 2 | ttgaggagacggataaagttgttgcactcg |
| SEQ ID NO: 3 | gcgacgttgcctgagattaagtgtccagtc |

Referring to FIG. 6, in each of the following embodiments, the nucleic acid extraction layer 2200 is a glass fiber membrane containing the lysis solution that is dried, wherein the lysis solution is bacterial cell lysis solution, but the practice is not limited thereto, and the nucleic acid extraction layer 2200 may be filter paper, gel, non-woven fabric, cellulose membrane, polyester film, polypropylene film, and the lysis solution may be adjusted according to the sample to be tested, so that the other types of lysis solution may also be used to lyse cells, viruses, fungi, protozoa, algae and other types of samples to be tested, so that the sample to be tested releases nucleic acid.

Referring to FIG. 6, in each of the following embodiments, the nucleic acid adsorption layer 2202 is silica membrane, but the practice is not limited thereto, the nucleic acid adsorption layer 2202 may also be film with silica microparticles, film with diatomaceous earth, filter paper, whatman 903 filter paper, cellulose membrane, Fusion 5 filter paper, polyethersulfone (PES) film, or nylon film.

In each of the following embodiments, the first cleaning liquid and the second cleaning liquid are both anhydrous ethanol, but the practice is not limited thereto, the first cleaning liquid and the second cleaning liquid may also be isopropanol or 95% V/V ethanol, and the first cleaning liquid and the second cleaning liquid may be different cleaning liquid, for example, the first cleaning liquid is isopropanol, and the second cleaning liquid is anhydrous ethanol.

Referring to FIG. 1, FIG. 4, FIG. 5, FIG. 6, and FIG. 7, in each of the following embodiments, the bearing body 2 is the aforementioned first form of the bearing body 2 of the nucleic acid detection device 1 as an example, the substrate part 20 is a rectangular sheet, and the bridge-shaped part 22 is a U-shaped sheet formed by the first sidewall 22a, the connecting face 22b and the second sidewall 22c of the bridge-shaped part 22 together, so that the channel 222 is formed between the substrate part 20 and the bridge-shaped part 22, the starting pad 30 of the detection body 3 is located in the channel 222, the connecting face 22b of the bridge-shaped part 22 is provided with the sample pad 220. Just below the sample pad 220 corresponds to the starting pad 30 of the detection body 3, and the other end of the detection body 3 passes through the second sidewall 22c via the window 224, and the bottom edge of the window 224 is the upper surface of the substrate part 20, so that the detection body 3 can be laid on the upper surface of the substrate part 20, so that the control line 340 and the test line 342 are located outside the channel 222, which is convenient for the user to observe, but the practice is not limited thereto, the shape of the substrate part 20 may also be other shapes, such as circle and square, which may be adjusted according to practical requirements, which is not specifically limited in the present invention, the bridge-shaped part 22 may also be an arc-shaped sheet formed by connecting the first sidewall 22a, the connecting face 22b and the second sidewall 22c of the bridge-shaped part 22 together, and in addition, other equivalent changes or modifications in forming a bridge-shaped structure and forming the channel 222 between the bridge-shaped structure and the substrate part 20 shall still fall within the scope of the present invention; wherein the material of the bridge-shaped part 22 is a white paperboard in a paper material, but the practice is not limited thereto, and may also be other types of paper materials, such as laminated paper, kraft paper, drawing paper and other paper materials, and the bridge-shaped part 22 may also be a flexible material, for example, flexible plastic, flexible glass or rubber, etc., wherein the flexible plastic is, for example, polyethelyne (PE), polyethylene terephthalate (PET). Since the bridge-shaped part 22 may be bent, after the motion body 4 is pulled away from the channel 222, the first sidewall 22a and the second sidewall 22c of the bridge-shaped part can be bent by pressing the connecting face 22b, or the connecting face 22b of the bridge is bent toward the starting pad 30, so that the sample pad 220 of the connecting face 22b is in contact with the starting pad 30 of the detection body 3; wherein the sample pad 220 is fixed to the connecting face 22b in an embedded manner, wherein the embedded manner is that a through hole is reserved at one end of the motion body 4, and the sample pad 220 is fixed in a through hole in the adhesive manner, and the upper surface and the lower surface of the sample pad 220 are exposed simultaneously, but the practice is not limited thereto, and the sample pad 220 can also be fixed in the through hole by using a double coated film, a foam adhesive, or using a clamp to fix; wherein the sample pad 220 includes the nucleic acid extraction layer 2200 and the nucleic acid adsorption layer 2202, wherein the nucleic acid extraction layer 2200 is stacked on the upper surface of the nucleic acid adsorption layer 2202.

Referring to FIG. 4, FIG. 6, and FIG. 7, in each of the following embodiments, the detection body 3 is lateral flow immunochromatography paper, and the detection body 3 is fixed on the upper surface of the bearing body 2 in an adhesive manner, wherein the detection body 3 includes the starting pad 30, the binding pad 32, the reaction film 34, and absorption pad 36, the starting pad 30 is provided at one end of the detection body 3, the absorption pad 36 is provided at the other end of the detection body 3, one end of the starting pad 30 is stacked at one end of the binding pad 32, the other end of binding pad 32 is stacked at one end of the reaction film 34, and one end of the absorption pad 36 is stacked at the other end of the reaction film 34; wherein the starting pad 30 is used for receiving the eluent and the amplicons of the target nucleic acid, and enabling the amplicons of the target nucleic acid to flow to the binding pad 32 along with the eluent, wherein since the nucleic acid adsorption layer 2202 is silica membrane in each of the following embodiments, the silica membrane adsorbs the amplicons of the target nucleic acid, so that the pH value or salt concentration of the silica membrane can be subsequently changed by the eluent, so that the amplicons of the target nucleic acid is released from the silica membrane, and as the eluent flows to the starting pad 30 of the detection body 3, wherein the eluent is nuclease-free water, but the practice is not limited thereto, and may also be a low-salt buffer, for example, a Tris-EDTA buffer (TE buffer) having the pH value of 8.0, and the TE buffer includes Tris(hydroxymethyl)aminomethane (Tris) and ethylenediaminetetraacetic acid (EDTA), wherein the concentration of the Tris(hydroxymethyl)aminomethane can be 10 mM, wherein the concentration of the ethylenediaminetetraacetic acid can be 1 mM, but the practice is not limited thereto, the concentration and pH value of each component in the low-salt buffer may be adjusted according to practical requirements, and is not limited to the TE buffer, which may also be other buffer used in eluting nucleic acid.

Referring to FIG. 4, in each of the following embodiments, the starting pad 30 is a glass fiber membrane, but the practice is not limited thereto and may be filter paper, non-woven fabric, cellulose membrane, polyester membrane, or polypropylene film.

Referring to FIG. 4, in each of the following embodiments, the binding pad 32 includes a chromogenic substance, wherein the binding pad 32 is a glass fiber membrane, wherein the chromogenic substance is a carrier particle with anti-chemical marker antibody, and includes an anti- chemical marker antibody and a carrier particle, wherein the anti-chemical marker antibody is connected to the surface of the carrier particle, wherein the anti-chemical marker antibody is anti-biotin antibody, wherein the carrier particle is a gold nanoparticle. Since the amplicons of the target nucleic acid is provided with the chemical marker, the anti-chemical marker antibody in the chromogenic substance will bind to the chemical marker on the target nucleic acid, but the practice is not limited thereto, the binding pad 32 may also be filter paper, non-woven fabric, cellulose membrane, polyester membrane, or polypropylene film; wherein the carrier particle in the chromogenic substance is not limited to gold nanoparticle, and may also be carrier such as fluorescent microbead, colored polystyrene microbead, magnetic microbead, carbon nanoparticle, selenium nanoparticle, and silver nanoparticle; wherein the surface of the carrier particle in the chromogenic substance is not limited to being connected to the anti-chemical marker antibody, and may also be anti-chemical marker nucleic acid aptamer, anti-fluorescent marker antibody, or anti-fluorescent marker nucleic acid aptamer; in another embodiment of the present invention, the chromogenic substance may also be organic fluorescent group or textile dye.

Referring to FIG. 4, in each of the following embodiments, the reaction film 34 is nitrocellulose membrane, and the reaction film 34 enables the eluent flowing to the binding pad 32 to move in the direction toward the absorption pad 36 by using capillary action, wherein the amplicons of the target nucleic acid and the chromogenic substance also move in the direction toward the absorption pad 36, but the practice is not limited thereto, the reaction film 34 may also be a poly(vinylidene fluoride) (PVDF), cellulose acetate, or nylon film.

Referring to FIG. 4, in each of the following embodiments, the number of the test lines 342 of the reaction film 34 is one, and the test line 342 includes the anti-fluorescent marker antibody, wherein the anti-fluorescent marker antibody is anti-fluorescein isothiocyanate antibody, and since the amplicons of the target nucleic acid is provided with the fluorescent marker, the amplicons of the target nucleic acid is captured by the anti-fluorescent marker antibody of the test line 342 and is fixed to the test line 32, and since the target nucleic acid is bound with the chromogenic substance, it forms a colored band that can be visually observed, but the practice is not limited thereto, the number of the test lines 342 of the reaction film 43 may also be multiple, and the test line 342 is not limited to anti-fluorescent marker antibody, and may also be adjusted according to the markers on the target nucleic acid to be captured, and is not limited to nucleic acid aptamer, for example, the anti-fluorescent marker antibody may be replaced by anti-chemical marker antibody, anti-fluorescent marker nucleic acid aptamer, or anti-chemical marker nucleic acid aptamer.

Referring to FIG. 4, in each of the following embodiments, the number of the control lines 340 of the reaction film 34 is one, and the control line 340 includes a secondary antibody, wherein the secondary antibody is an antibody for capturing the anti-chemical marker antibody in the chromogenic substance, so that the chromogenic substance is fixed to the control line 340, forming a colored band that can be visually observed, but the practice is not limited thereto, the number of the control lines 340 of the reaction film 43 may also be multiple, and the secondary antibody on the control line 340 of the reaction film 34 may be adjusted according to the form of the chromogenic substance to be captured, for example, may be anti-fluorescent marker antibody, anti-chemical marker nucleic acid aptamer, or anti-fluorescent marker nucleic acid aptamer.

Referring to FIG. 4, in each of the following embodiments, the absorption pad 36 is high absorption paper, wherein the high absorption paper such as filter paper, is used to absorb the eluent flowing from the reaction film 34 to the absorption pad 36, but the practice is not limited thereto, the absorption pad 36 may also be a high absorption material, such as non-woven fabric and absorbent gel.

Referring to FIG. 1, FIG. 3, FIG. 5, and FIG. 6, in each of the following embodiments, the motion substrate 40 of the motion body 4 is a rectangular sheet, and the motion substrate 40 is provided with two liquid absorption pads and one waterproof pad WP, in the following, the two liquid absorption pads are respectively referred to as a first liquid absorption pad AP1 and a second liquid absorption pad AP2, the waterproof pad WP is disposed between the first liquid absorption pad AP1 and the second liquid absorption pad AP2, and the area where the first liquid absorption pad AP1 is disposed and the area where the second liquid absorption pad AP2 is disposed on the motion body 4 are liquid absorption areas, and the area where the waterproof pad WP is disposed on the motion body 4 is non-liquid absorption area B, wherein the area on the motion body 4 where the first liquid absorption pad AP1 is disposed is referred to as a first liquid absorption area A1, the area on the motion body 4 where the second liquid absorption pad AP2 is disposed is referred to as a second liquid absorption area A2, the liquid absorption pads waterproof pad WP are both provided on the upper surface of the motion body 4 in an adhesive manner, the motion body 4 is detachably provided in the channel 222, and since the detection body 3 is disposed on the upper surface of the substrate part 20, a part of the motion body 4 located in the channel 222 is stacked on the upper surface of the detection body 3. When the motion body 4 is detachably provided in the channel 222, the motion body 4, the motion body 4 may move along the direction of the channel 222, enabling the first liquid absorption area A1, the non-liquid absorption area B, or the second liquid absorption area A2 of the motion body 4 to correspond to the sample pad 220, that is, the sample pad 220 is stacked on the upper surface of the first liquid absorption pad AP1, the upper surface of the waterproof pad WP, or the upper surface of the second liquid absorption pad AP2, and the motion body 4 may also be pulled away from the channel 222; wherein the length of the motion body 4 is greater than the length of the channel 222, thus when motion body 4 moves in the channel 222, only a part of the motion body 4 is accommodated in the channel 222, which is convenient for the user to move motion body 4 by grasping the part of the motion body 4 outside the channel in a hand pinching manner or in a pinching manner with using clamping tool. Since the sample pad 220 is fixed on the connecting face 22b of the bridge-shaped part 22, when the sample to be tested, the first cleaning liquid, the nucleic acid amplification reagent, or the second cleaning liquid is added, the sample pad 220 is maintained in the fixed position, and by moving the motion body 4 to make the liquid absorption area or non-liquid absorption area B corresponds to the sample pad 220, then the sample pad 220 can be directly added the sample to be tested, the first cleaning liquid, the nucleic acid amplification reagent, or the second cleaning liquid, compared with the structural design that the sample pad 220 needs to be moved to correspond to the liquid absorption area or non-liquid absorption area B, the present invention can reduce the burden of the user needing to continuously recheck the current position of the sample pad 220, so that achieving the effect of reducing the chance that the user mistakes adding position when adding the sample to be tested, the first cleaning liquid, the nucleic acid amplification reagent, or the second cleaning liquid.

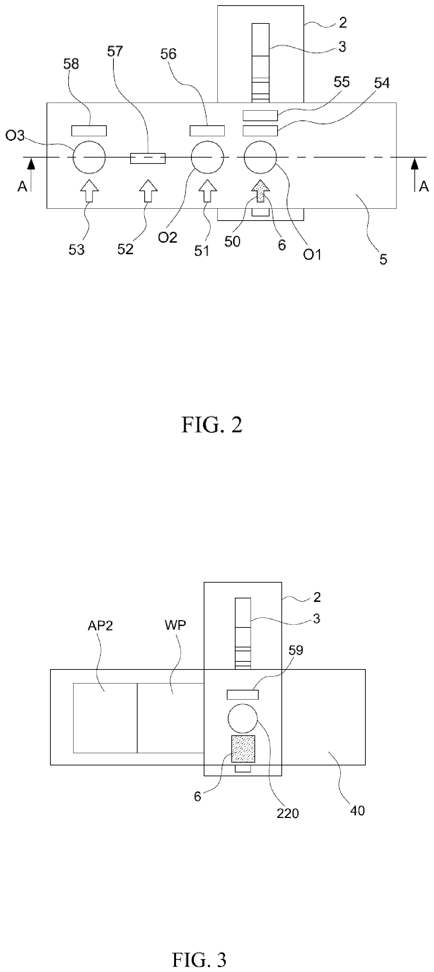

Referring to FIG. 1, FIG. 2, FIG. 3, FIG. 5, FIG. 6, and FIG. 11, in each of the following embodiments, the motion body 4 includes guide body 5, the guide body 5 is a rectangular sheet, one end of the motion body 4 is adhered to one end of the motion body 4, the plurality of openings of the guide body 5 includes a first opening O1, a second opening O2, and a third opening O3, wherein directly below the first opening O1 corresponds to the first liquid absorption area A1 of the motion body 4, wherein directly below the second opening O2 corresponds to a part of the non-liquid absorption area B of the motion body 4, wherein directly below the third opening O3 corresponds to the second liquid absorption area A2 of the motion body 4; the guide body 5 further includes the plurality of cue marks, and the bridge-shaped part 22 further includes guide mark 6, wherein the plurality of cue marks contains a first pattern mark 50, a second pattern mark 51, a third pattern mark 52, a fourth pattern mark 53, a first text mark 54, a second text mark 55, a third text mark 56, a fourth text mark 57, a fifth text mark 58, and a sixth text mark 59, wherein the first pattern mark 50, the second pattern mark 51, the third pattern mark 52, and the fourth pattern mark 53 are all arrow patterns marked on the guide body in a punching manner; wherein the first pattern mark 50 points to the first opening O1, the second pattern mark 51 points to the second opening O2, the fourth pattern mark points to the third opening O3. Wherein, the first text mark 54 and the second text mark 55 are marked beside the first opening O1, the third text mark 56 is marked beside the second opening O2, and a fifth mark 58 is marked beside the third opening O3. Wherein the fourth text mark 57 is marked at a position on the guide body 5 corresponding to another part of the non-liquid absorption area B of the motion body 4, and the third pattern mark 52 points to the fourth text mark 57. Wherein the sixth text mark 59 is marked beside the sample pad 220 of the connecting face 22b of the bearing body 2. Wherein the first text mark 54, the second text mark 55, the third text mark 56, the fourth text mark 57, the fifth text mark 58 and the sixth text mark 59 are all represented by text boxes in FIG. 1 and FIG. 2, the text filled in each text box in FIG. 1 and FIG. 2 are described below, wherein the text of the first text mark 54 is "Step 1: adding a sample to be tested", wherein the text of the second text mark 55 is "Step 2: adding a first cleaning liquid", wherein the third text mark 56 is "Step 3: adding a nucleic acid amplification reagent", wherein the text of the fourth text mark 57 is "Step 4: waiting for nucleic acid amplification", wherein the text of the fifth text mark 58 is "Step 5: pulling away a motion body after adding a second cleaning liquid", wherein the sixth text mark 58 is "Step 6: adding an eluent after pressing", wherein the first text mark 54 is a hint to carry out sample adding step S20, wherein the second text mark 55 is a hint to carry out the first cleaning step S25, wherein the third text mark 56 and fourth text mark 57 are a hint to carry out the nucleic acid amplification step S30, wherein the fifth text mark 58 is a hint to carry out the second cleaning step S35, wherein the sixth text mark 59 is a hint to carry out the nucleic acid detection step S40. The user can pinch an end where the motion body 4 and the guide body 5 adhered to each other by the hand, and move the motion body 4 along the direction of the channel 222 by pushing or pulling the motion body 4, or pulling the motion body 4 away from the channel 222, and in addition, when the liquid absorption area or non-liquid absorption area B of the motion body 4 corresponds to the sample pad 220, the finger or the clamping tool further presses the connecting face 22b to strengthen the contact between the sample pad 220 of the connecting face 22b and the liquid absorption area or non-liquid absorption area B of the motion body 4, and then the sample to be tested, the first cleaning liquid, the nucleic acid amplification reagent or the second cleaning liquid is added, thereby stabilizing the position of the sample pad 220, and ensuring the liquid absorption area can completely absorb the residual liquid by making the sample pad 220 in sufficient contact with the liquid absorption area. The aforementioned examples are merely exemplary, and the practice is not limited thereto, in another embodiment of the present invention, the sample pad 220 may include only the nucleic acid adsorption layer 2202, and the at least one opening of the plurality of openings corresponding to the liquid absorption area of the motion body 4 is provided with the nucleic acid extraction layer 2200, for example, the nucleic acid extraction layer 2200 is disposed in the first opening O1, wherein the nucleic acid extraction layer 2200 may be in an adhesive manner to fix the nucleic acid extraction layer 2200 in the first opening O1 and expose the upper surface and the lower surface of the nucleic acid extraction layer 2200, thus when the first opening O1 corresponds to the sample pad 220, the lower surface of the nucleic acid extraction layer 2200 in the first opening O1 is in contact with the upper surface of the nucleic acid adsorption layer 2202. The aforementioned examples are merely exemplary, and the practice is not limited thereto, other equivalent changes or modifications made to the aforementioned content of the present invention shall still fall within the scope of the present invention.

### Embodiment 1: Evaluating the working temperature, the working time, and the nucleic acid detection range of the nucleic acid detection method provided by the present invention by using the Agrobacterium genomic nucleic acid

In Embodiment 1, the Agrobacterium genomic nucleic acid was used as an example of the sample to be tested, and in Embodiment 1, the nucleic acid adsorption layer 2202 was used as an independent component to perform nucleic acid amplification test and was coupled with the detection body 3 for nucleic acid amplification test.

In Embodiment 1, the nucleic acid amplification was to add a nucleic acid amplification reagent to nucleic acid adsorption layer 2202 to form the amplicons of the target nucleic acid and a multi-temperature heating and cooling shaker was used as a temperature control device during the nucleic acid amplification to control the temperature for the nucleic acid amplification, and measure and record the reaction temperature by using the handheld temperature data logger.

The experimental steps of Embodiment 1 were as follows: firstly, placing the nucleic acid adsorption layer 2202 on a culture dish, then placing the culture dish on multi-temperature heating and cooling shaker, and then adding the sample to be tested to the nucleic acid adsorption layer 2202, so that the nucleic acid in the sample to be tested was adsorbed by the nucleic acid adsorption layer 2202, then letting the nucleic acid adsorption layer 2202 to stand to wait for the nucleic acid adsorption layer 2202 to dry naturally, then adding the nucleic acid amplification reagent to the nucleic acid adsorption layer 2202, and then controlling the temperature for the nucleic acid amplification by multi-temperature heating and cooling shaker, so that the nucleic acid amplification is performed on the target nucleic acid in the nucleic acid adsorption layer 2202 to form the amplicons of the target nucleic acid, and at the same time, during the nucleic acid amplification, after the temperature of nucleic acid amplification was measured and recorded by the handheld temperature data logger, and then after the nucleic acid amplification was completed, the nucleic acid adsorption layer 2202 was placed to the liquid absorption area of the motion body 4 by tweezers, then the second cleaning liquid was added to the nucleic acid adsorption layer 2202 to wash away the nucleic acid amplification reagent remaining in the nucleic acid adsorption layer 2202, and then the nucleic acid adsorption layer 2202 was let to stand to wait for the nucleic acid adsorption layer 2202 to dry naturally, then after the nucleic acid adsorption layer 2202 was placed to the starting pad 30 of the motion body 4 by tweezers, and then the eluent was added to the nucleic acid adsorption layer 2202, so that the amplicons of the target nucleic acid in the nucleic acid adsorption layer 2202 flowed to the starting pad 30 along with the eluent, then flowed through the binding pad 32 form the starting pad 30, and then reacted with the reaction film 34, wherein the amplicons of the target nucleic acid was captured by the test line 342; in the following, the temperature for the nucleic acid amplification is referred to as the "working temperature", and the time for the nucleic acid amplification is referred to as the "working time". Wherein the samples to be tested were tested at 10⁶ copy number, 10⁴ copy number, 10² copy number, 10 copy number, 1 copy number, and 0 copy number of the Agrobacterium genomic nucleic acid respectively, the working temperatures were tested at 20°C., 23°C., 25°C., 30°C., 35°C., 40°C., 45°C and 50°C respectively, and the working time was tested at 5 minutes, 15 minutes, 25 minutes, 35 minutes, 45 minutes and 55 minutes, respectively. Each experimental condition was performed in triplicate, and the test result was judged to be positive result when both the test line 342 and the control line 340 on the each detection body 3 in triplicate experiments all had a positive reaction, and the positive reaction was judged to have successfully detected the target nucleic acid when both the test line 342 and the control line 340 of the detection body 3 showed color development, and if the detection body 4 in any one experiment of the triplicate experiments only had the color development on the control line 340 and no color development on the test line 342, it was judged that the detection result was a negative result, wherein under the same test condition, if it was judged to be the positive result, that is, all triplicate experiments showed positive reaction, and denoted by "P", and separated by a comma with the value of the minimum copy number of the Agrobacterium genomic nucleic acid required for the positive result, and if each group showed the negative result, it was denoted by "N". The experimental results of Embodiment 1 are shown in Table 2 below.

In Table 2 below, the vertical columns represent different working temperatures, and the horizontal rows represent different working times, for example, as shown in Table 2, the intersection of the vertical column of 5 minutes and the horizontal row of 35°C is shown as "P, 10⁶", which represents that under the condition that the working time was 5 minutes and the working temperature was 35°C., the detection body 3 in the triplicate experiments all showed the positive reaction, so it was judged as the positive result, and the minimum copy number of the Agrobacterium genomic nucleic acid required 10⁶ copy number, which meant that the positive result was obtained only when the sample to be tested was 10⁶ copy number of the Agrobacterium genomic nucleic acid, and the negative result would be obtained when the sample to be tested was 10⁴ copy number, 10² copy number, 10 copy number, 1 copy number, and 0 copy number of the Agrobacterium genomic nucleic acid; in addition, for example, as shown in Table 2, the intersection of the vertical column of 15 minutes and the horizontal row of 30°C is shown as "P, 10", which represents that under the condition that the working time was 15 minutes and the working temperature was 30°C., the detection body 3 in the triplicate experiments all showed the positive reaction, so it was judged as the positive result, and since the minimum copy number of the Agrobacterium genomic nucleic acid required 10 copy number, which meant that the positive result could be obtained when the sample to be tested was 10 copy number of the Agrobacterium genomic nucleic acid, the positive result would be also obtained when the sample to be tested is the Agrobacterium genomic nucleic acid with a higher copy number, that is, the positive result would be also obtained when the sample to be tested is 10⁶ copy number, 10⁴ copy number, 10² copy number of the Agrobacterium genomic nucleic acid, and the negative result would be obtained when the sample to be tested is 1 copy number and 0 copy number; the above are two examples, and so on.

The experimental results of Table 2 below show that when the working temperature was 23°C and the working time was 45 minutes, the sample to be tested needed at least 10⁴ copy number of the Agrobacterium genomic nucleic acid to obtain the positive result, that is, it represented that triplicate experiments were all positive reaction. When the working temperature was 23°C and the working time is 55 minutes, the target nucleic acid could be detected only by 10² copy number of the Agrobacterium genomic nucleic acid of the sample to be tested, it was shown that at the same working temperature, the extended working time helped to detect a lower copy number of the Agrobacterium genomic nucleic acid, that is, the sensitivity of detecting the target nucleic acid was improved; in addition, when the working time is fixed to 55 minutes, the Agrobacterium genomic nucleic acid could be detected at working temperature 23-50°C, wherein when the working temperature was 23°C., the Agrobacterium genomic nucleic acid with 10² copy number could be detected, and when the temperature was 25 -50°C., the target nucleic acid could be detected only by 1 copy number of the Agrobacterium genomic nucleic acid of the sample to be tested, it represented that the sensitivity of detecting the target nucleic acid could also be improved with the increase of the working temperature at the same working time; besides, with the increase of the working temperature, the nucleic acid amplification could be accelerated, for example, when the sample to be tested was 1 copy number of the Agrobacterium genomic nucleic acid, the positive result would be observed for 45 minutes when the working temperature was 25°C., but it only took 25 minutes to observe the positive result when the working temperature was 30°C., and it only took 15 minutes to observe the positive result when the working temperature was 35°C. or 40°C., moreover, it only took 5 minutes to observe the positive result when the working temperature was 45°C. The experimental results of Embodiment 1 showed that the working temperature was 23-50°C., the working time was 5-55 minutes to obtain the amplicons of the target nucleic acid, and the target nucleic acid in 1 copy number of the Agrobacterium genomic nucleic acid could be detected, and thus further verification would be performed based on the experimental results; it was further noted that the aforementioned working temperature range and the working time range were not intended to limit the present invention, the working temperature range and the working time range in practice could be adjusted according to the nucleic acid amplification reagent.

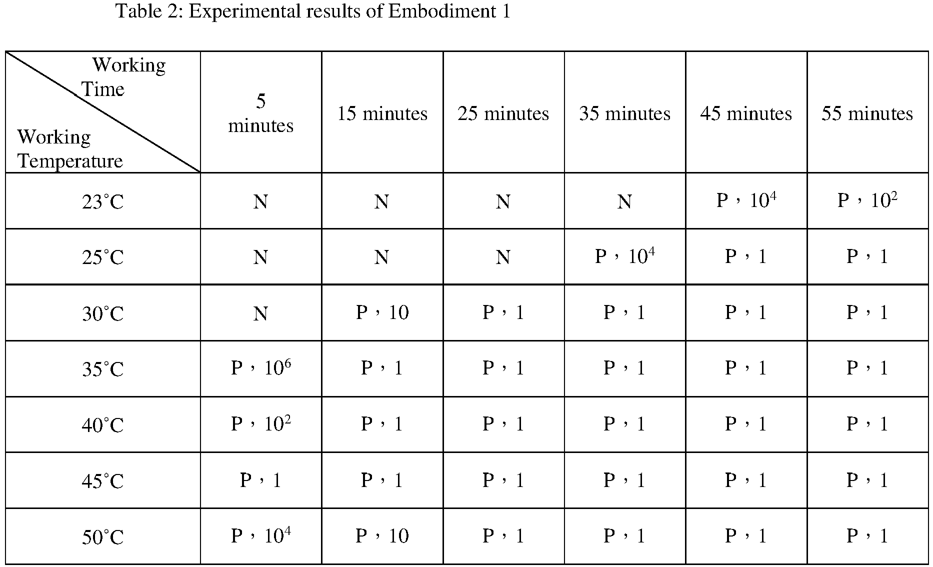

### Embodiment 2: Using the nucleic acid of the Agrobacterium to verify the method of the present invention can be performed at room temperature environment

The experimental steps of Embodiment 2 are basically the same as the experimental steps of Embodiment 1, differing only in that the nucleic acid amplification was changed to a room temperature environment, therefore the working temperature was the room temperature when the nucleic acid amplification was performed, the working time was fixed to 45 minutes, and a triplicate experiments was performed, wherein the working temperature the temperature of the nucleic acid amplification was also measured and recorded by the handheld temperature data logger, and the experimental results is shown in Table 3, Table 3 shows the measured working temperature for each of the triplicate experimental results, and 10⁶ copy number, 10⁴ copy number, 10² copy number, 10 copy number, 1 copy number, and 0 copy number of the Agrobacterium genomic nucleic acid were used as the sample to be tested, whether the detection body 3 presented the positive reaction, and if it showed the positive reaction, it was denoted by "O", while the negative was denoted by "X", wherein the group of 0 copy number of the Agrobacterium genomic nucleic acid was used as a negative control group. The experimental results showed that when the sample to be tested was 10 copy number of the Agrobacterium genomic nucleic acid, although the positive reaction was not obtained in the second test, the positive reaction was presented in the first test and the third test, and when the sample to be tested was 10² copy number of the Agrobacterium genomic nucleic acid, the triplicate experimental results all showed positive reaction, that is, the positive result was judged, and this result was similar to the result in Embodiment 1 in which the target nucleic acid of 1 copy number of the Agrobacterium genomic nucleic acid of the sample to be tested could be detected when the working temperature was 25°C., and working time was 45 minutes, therefore the experimental results of Embodiment 2 showed that the nucleic acid amplification could also be accomplished without relying on the temperature control device to control the working temperature and placed in the room temperature environment where the temperature variations, and the sensitivity of detecting the target nucleic acid was equivalent to the effect of using the temperature control device. It is further noted that the aforementioned working temperature range and the working time range are not intended to limit the present invention, and since Embodiment 2 had proved that the nucleic acid amplification could be completed without temperature control, and Embodiment 1 proved that the nucleic acid amplification could be shortened along with the increase of the working temperature, so that the user could also use a heating product for home use to assist in heating, wherein the heating product for home use such as hand warmer and steam eye mask, for example, during the execution of the nucleic acid amplification step S30, the substrate part 20 is stacked on the hand warmer, so that heat emitted by the hand warmer is transferred to the sample pad 220, thereby increasing the speed of the nucleic acid amplification and shortening the required working time. It is further noted that the aforementioned working temperature range and the working time range are also not intended to limit the present invention.

**Table 3: Experimental results of Embodiment 2**

| Number of Experiments | Working Temperature (°C) | Agrobacterium Genomic Nucleic Acid (copy number) | | | | | |
|---|---|---|---|---|---|---|---|
| | | 10⁶ | 10⁴ | 10² | 10 | 1 | 0 |
| First | 24.6~24.8 | O | O | O | O | O | X |
| Second | 24.6-24.8 | O | O | O | O | X | X |
| Third | 24.8-25.0 | O | O | O | O | O | X |

Embodiment 3: Using the Agrobacterium to verify the method of the present invention can realize nucleic acid extraction, amplification, and detection of samples

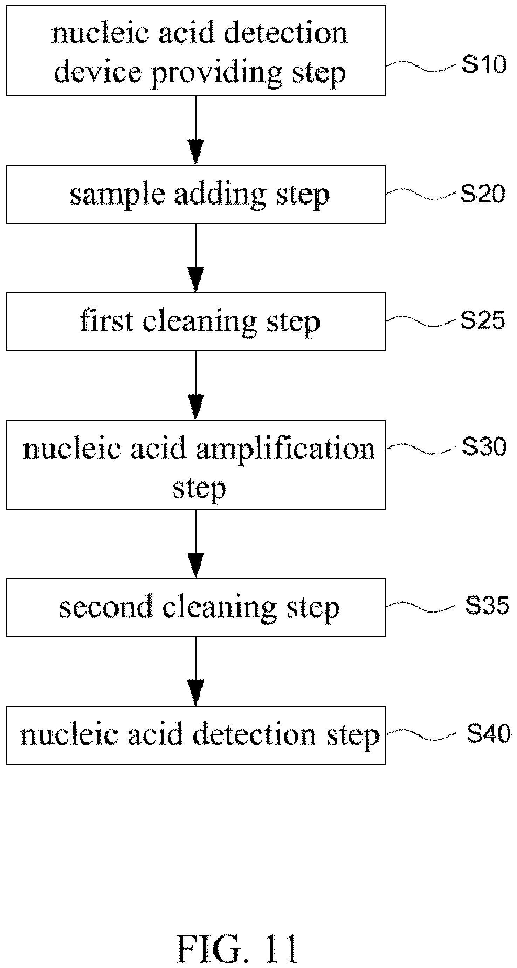

Referring to FIG. 11, the experimental method of Embodiment 3 was the nucleic acid detection method provided according to the present invention, and the nucleic acid detection method included the first cleaning step S25 and the second cleaning step S35, therefore the steps sequentially included the nucleic acid detection device providing step S10, the sample adding step S20, the first cleaning step S25, the nucleic acid amplification step S30, the seconds cleaning step S35, and the nucleic acid detection step S40, wherein the sample to be tested was an Agrobacterium suspension, and the concentration of the Agrobacterium in the Agrobacterium suspension was represented by colony forming unit (CFU) in 100 µL, and therefore the unit was CFU/100 µL, wherein the CFU was calculated by applying an appropriate diluted 100 µL of the Agrobacterium suspension to a culture medium, forming single colony on the culture medium, wherein each colony forming unit is referred as 1 CFU, and the concentration of the Agrobacterium in the Agrobacterium suspension in Embodiment 3 was 1.5×10⁶ CFU/100 µL , 1.5×10⁴ CFU/100 µL , 150 CFU/100 µL , 15 CFU/100 µL , 1.5 CFU/100 µL, and 0 CFU/100 µL, respectively, and the following statements were provided with respect to each step of the nucleic acid detection method in paragraphs, and further specific descriptions were provided with reference to FIG. 1, FIG. 2, FIG. 3, FIG. 4, FIG. 5, FIG. 6, FIG. 7, and FIG. 11.

The nucleic acid detection device providing step S10 was to provide the nucleic acid detection device 1 of the present invention, wherein the specific form of the nucleic acid detection device 1 was as described above.

The sample adding step S20 was to make the liquid absorption area of the motion body 4 correspond to the sample pad 220, and then add the sample to be tested to the sample pad 220, wherein the sample to be tested included the target nucleic acid; in Embodiment 3, specifically, the user moved the motion body 4 and the guide body 5 simultaneously according to the instructions of the first text mark 54, so that the first pattern mark 50 overlapped with the guide mark 6, and at this time, the sample pad 220 corresponded to the first opening O1, and the sample pad 220 also corresponded to the first liquid absorption area A1,wherein since the sample to be tested was the Agrobacterium suspension, thus after the Agrobacterium suspension was added to the sample pad 220, the Agrobacterium of the Agrobacterium suspension would be first lysed by the lysis solution in nucleic acid extraction layer 2200, and the nucleic acid of the Agrobacterium would be exposed, and then the nucleic acid of the Agrobacterium would be adsorbed by the nucleic acid adsorption layer 2202, and the residual liquid would be absorbed by the first absorption area A1.

The first cleaning step S25 was to make the liquid absorption area of the motion body 4 correspond to the sample pad 220, and then add the first cleaning liquid to the sample pad 220; in Embodiment 3, specifically, the user maintained the sample pad 220 corresponding to the first opening O1 according to the instructions of the second text mark 55, at the same time, the sample pad 220 also corresponded to the first liquid absorption area A1, and then the first cleaning liquid was added to the sample pad 220, so that the impurities in the sample pad 220 flowed out along with the first cleaning liquid and were absorbed in the first liquid absorption area A1.

The nucleic acid amplification step S30 was to make the non-liquid absorption area B of the motion body 4 corresponded to the sample pad 220, and then make the target nucleic acid in the sample pad 220 to react to the nucleic acid amplification reagent to form the amplicons of the target nucleic acid; in Embodiment 3, specifically, the user moved the motion body 4 and the guide body 5 simultaneously according to the instructions of the third text mark 56, so that the second pattern mark 51 overlapped with the guide mark 6, and the sample pad 220 also corresponded to the second opening O2, at the same time the sample pad 220 also corresponded to the non-liquid absorption area B of the motion body 4, then the nucleic acid amplification reagent was added to the sample pad 220, and then the motion body 4 and guide body 5 were moved simultaneously according to the instructions of the fourth text mark 57, so that the third pattern mark 52 was overlapped with the guide mark 6, at this time, the sample pad 220 was covered by the guide body 5, at the same time, the sample pad 220 also corresponded to the non-liquid absorption area, and then waited for performing the nucleic acid amplification according to the fourth text mark 57, in this embodiment, the working time was 45 minutes for nucleic acid amplification, so that the target nucleic acid in the sample pad 220 fully reacted with the nucleic acid amplification reagent to form the amplicons of the target nucleic acid, and the amplicons of the target nucleic acid was adsorbed by the nucleic acid adsorption 2202.

The second cleaning step S35 was to make the liquid absorption area of the motion body 4 correspond to the sample pad 220, and then add the second cleaning liquid to the sample pad 220; in Embodiment 3, specifically, the user made the motion body 4 and guide body 5 were moved simultaneously according to the instructions of the fifth text mark 58, so that the fourth pattern mark 53 was overlapped with the guide mark 6, at this time, the sample pad 220 corresponded to the third opening O3, at the same time, the sample pad 220 also corresponded to the liquid absorption pad of the second liquid absorption area A2, and then the second cleaning liquid was added to the sample pad 220, so that the impurities in the sample pad 220 flowed out along with the second cleaning liquid and were absorbed in the second liquid absorption area A2.

In the nucleic acid detection step S40, after the motion body 4 was pulled away from the channel 222, the bridge-shaped part 22 was pressed to bend the bridge-shaped part 22, and the sample pad 220 was in contact with the starting pad 30 of the detection body 3, and then the eluent was added to the sample pad 220, so that the amplicons of the target nucleic acid in the sample pad 220 flowed to the starting pad 30 of the detection body 3 along with the eluent, and then flowed to the at least one control line 340 of the detection body 3 and the at least one test line 342 from the starting pad 30, wherein the amplicons of the target nucleic acid would be captured by the at least one test line 342; in Embodiment 3, specifically, the user pulled the motion body 4 and the guide body 5 away, and after the guide body 5 left the upper surface of the connecting face 22b of the bearing body 2, since the bridge-shaped part 22 could be bent, so that the sample pad 220 could be pressed by finger to contact with the starting pad 30, and then the eluent was added to the sample pad 220, so that the amplicons of the target nucleic acid in the sample pad 220 flowed to the starting pad 30 of the detection body 3 along with the eluent, and then the starting pad 30 flowed through the binding pad 32, so that the chromogenic substance in the binding pad 32 was bound to the amplicons of the target nucleic acid, and then the amplicons bound with the chromogenic substance was captured by the test line 342 to form the colored band which could be visually observed, so that the effect of detecting the target nucleic acid in the sample to be tested was achieved, the unreacted chromogenic substance was captured by the control line 340, and the residual liquid was finally absorbed by the absorption pad 36.

The experimental results of Embodiment 3 are shown in Table 4, Table 4 presents the working temperature measured in each of the triplicate experimental results, and whether the detection body 3 showed the positive reaction when the Agrobacterium was used as the sample to be tested, and if the positive reaction was shown, it was denoted by "O", while the negative was denoted by by "X", wherein the concentration of the Agrobacterium in the Agrobacterium suspension was 1.5×10⁶ CFU/100 µL , 1.5×10⁴ CFU/100 µL , 150 CFU/100 µL , 15 CFU/100 µL , 1.5 CFU/100 µL , 0 CFU/100 µL, respectively, wherein the concentration of the Agrobacterium in the Agrobacterium suspension was 0 CFU/100 µL as a negative control group; the experimental results showed that when the concentration of the Agrobacterium in the Agrobacterium suspension was 1.5 CFU/100 µL, the results of the triplicate experiments all presented the positive reaction, that is the positive result was obtained, and the triplicate experiments all presented the negative reaction in the group in which the concentration of the Agrobacterium in the Agrobacterium suspension was 0 CFU/100 µL, it represented that the nucleic acid detection method of the present invention did not generate a false positive reaction, which proves that the nucleic acid detection device 1 and the nucleic acid detection method of the present invention, without controlling the temperature, did complete the nucleic acid amplification of the target nucleic acid, and obtained the amplicons of the target nucleic acid, and successfully detected the amplicons of the target nucleic acid.

**Table 4: Experimental results of Embodiment 3**

| Number of Experiments | Working Temperature (°C) | Agrobacterium Genomic Nucleic Acid (CFU/100 µL) | | | | | |
|---|---|---|---|---|---|---|---|
| | | 1.5×10⁶ | 1.5×10⁴ | 150 | 15 | 1.5 | 0 |
| First | 25.8~25.9 | O | O | O | O | O | X |
| Second | 25.8-25.9 | O | O | O | O | O | X |
| Third | 25.8-25.9 | O | O | O | O | O | X |

In summary, the nucleic acid detection device and nucleic acid detection method provided by the present invention can complete nucleic acid amplification of the target nucleic acid in the sample to be tested at room temperature without temperature control, so as to obtain the amplicons of the target nucleic acid, and verify the presence of the target nucleic acid by detecting the amplicons, and meanwhile, through the structure of the nucleic acid detection device provided by the present invention, it is convenient for the user to complete the process from the sample adding step, the nucleic acid amplification step to the nucleic acid detection step in the home environment, which solves the problem that currently lacking a nucleic acid detection device that can be used at home and has nucleic acid amplification function and nucleic acid detection function.

Each embodiment is merely used to illustrate the content of the present invention but is not intended to limit the practice scope of the present invention, and therefore, all changes or modifications made according to the claims of the present invention shall still fall within the scope of the present invention.

## Claims

**1.** A nucleic acid detection device, comprising a bearing body, a detection body, and a motion body, wherein the bearing body comprises a substrate part and bridge-shaped part; wherein the bridge-shaped part is disposed on the upper surface of the substrate part, and a channel is formed between the bridge-shaped part and the substrate part; wherein the detection body comprises a starting pad, at least one control line, and at least one test line; wherein the detection body is disposed on the upper surface of the substrate part, and the starting pad of the detection body is located in the channel; wherein the bridge-shaped part is provided with a window for viewing a detection result shown by the at least one control line and the at least one test line of the detection body;

**2.** The nucleic acid detection device as claimed in claim 1, wherein the sample pad comprises a nucleic acid adsorption layer.

**3.** The nucleic acid detection device as claimed in claim 2, wherein the sample pad further comprises a nucleic acid extraction layer, and the nucleic acid extraction layer is disposed on the upper surface of the nucleic acid adsorption layer.

**4.** The nucleic acid detection device as claimed in claim 1, wherein the sample pad further comprises a nucleic acid amplification reagent.

**5.** The nucleic acid detection device as claimed in claim 1, wherein further comprises a guide body, one end of the guide body is connected to the upper surface of the motion body, and the guide body covers the upper surface of the sample pad of the bridge-shaped part of the bearing body; wherein a plurality of openings is provided on the guide body, wherein at least one of the plurality of openings corresponds to the liquid absorption area, and at least one of the plurality of openings corresponds to the non-liquid absorption area.

**6.** The nucleic acid detection device as claimed in claim 5, wherein at least one of the openings of the plurality of the openings corresponding to the liquid absorption area of the motion body is further provided with a nucleic acid extraction layer.

**7.** A nucleic acid detection method, comprising the following steps in sequence:
a nucleic acid detection device providing step: providing the nucleic acid detection device as claimed in claim 1;
a sample adding step: after the liquid absorption area of the motion body corresponds to the sample pad, adding a sample to the tested to the sample pad, wherein the sample to be tested comprises a target nucleic acid;
a nucleic acid amplification step: after the non-liquid absorption area of the motion body corresponds to the sample pad, enabling the target nucleic acid in the sample pad to react with a nucleic acid amplification reagent to form amplicons of the target nucleic acid;
a nucleic acid detection step: after the motion body is pulled away from the channel, pressing the bridge-shaped part to make the sample pad on the to contact with the starting pad of the detection body, and then adding an eluent to the sample pad, so that the amplicons of the target nucleic acid in the sample pad flow to the starting pad of the detection body along with the eluent, and then flow to the at least one control line and the at least one test line of the detection body from the starting pad, wherein the amplicons of the target nucleic acid is captured on the at least one test line.

**8.** The nucleic acid detection method as claimed in claim 7, wherein after completing the sample adding step, further comprises a first cleaning step, wherein the first cleaning step is to add a first cleaning liquid to the sample pad after the liquid absorption area of the motion body corresponds to the sample pad.

**9.** The nucleic acid detection method as claimed in claim 7, wherein after completing the nucleic acid amplification step, further comprises a second cleaning step, wherein the second cleaning step is to add a second cleaning liquid to the sample pad after of the liquid absorption area of the motion body corresponds to the sample pad.

**10.** The nucleic acid detection method as claimed in claim 7, wherein after completing the sample adding step, further comprises a first cleaning step, and after completing the nucleic acid amplification step, further comprises a second cleaning step; wherein the first cleaning step is to add a first cleaning liquid to the sample pad after the liquid absorption area of the motion body corresponds to the sample pad; wherein the second cleaning step is to add a second cleaning liquid to the sample pad after the liquid absorption area of the motion body corresponds to the sample pad.

## Amended claims

### Amended claims under Art. 19.1 PCT

**1.** A nucleic acid detection device, comprising a bearing body, a detection body, and a motion body, wherein the bearing body comprises a substrate part and bridge-shaped part; wherein the bridge-shaped part is disposed on the upper surface of the substrate part, and a channel is formed between the bridge-shaped part and the substrate part; wherein the detection body comprises a starting pad, at least one control line, and at least one test line; wherein the detection body is disposed on the upper surface of the substrate part, and the starting pad of the detection body is located in the channel; wherein the bridge-shaped part is provided with a window for viewing a detection result shown by the at least one control line and the at least one test line of the detection body; wherein a sample pad is provided at a position, corresponding to the starting pad of the detection body, of the bridge-shaped part; wherein the motion body is detachably provided in the channel, and the motion body comprises at least one liquid absorption area and at least one non-liquid absorption area; wherein the bridge-shaped part and the substrate part jointly limit the moving position of the motion body, and allowing the motion body to move in the direction of the channel, so that the liquid absorption area of the motion body corresponds to the sample pad, or the non-liquid absorption area of the moving body to correspond to the sample pad.

**2.** The nucleic acid detection device as claimed in claim 1, wherein the sample pad comprises a nucleic acid adsorption layer.

**3.** The nucleic acid detection device as claimed in claim 2, wherein the sample pad further comprises a nucleic acid extraction layer, and the nucleic acid extraction layer is disposed on the upper surface of the nucleic acid adsorption layer.

**4.** The nucleic acid detection device as claimed in claim 1, wherein the sample pad further comprises a nucleic acid amplification reagent.

**5.** The nucleic acid detection device as claimed in claim 1, wherein further comprises a guide body, one end of the guide body is connected to the upper surface of the motion body, and the guide body covers the upper surface of the sample pad of the bridge-shaped part of the bearing body; wherein a plurality of openings is provided on the guide body, wherein at least one of the plurality of openings corresponds to the liquid absorption area, and at least one of the plurality of openings corresponds to the non-liquid absorption area.

**6.** The nucleic acid detection device as claimed in claim 5, wherein at least one of the openings of the plurality of the openings corresponding to the liquid absorption area of the motion body is further provided with a nucleic acid extraction layer.

**7.** A nucleic acid detection method, comprising the following steps in sequence:
a nucleic acid detection device providing step: providing the nucleic acid detection device as claimed in claim 1;
a sample adding step: after the liquid absorption area of the motion body corresponds to the sample pad, adding a sample to the tested to the sample pad, wherein the sample to be tested comprises a target nucleic acid;
a nucleic acid amplification step: after the non-liquid absorption area of the motion body corresponds to the sample pad, enabling the target nucleic acid in the sample pad to react with a nucleic acid amplification reagent to form amplicons of the target nucleic acid;
a nucleic acid detection step: after the motion body is pulled away from the channel, pressing the bridge-shaped part to make the sample pad on the bridge-shaped part to contact with the starting pad of the detection body, and then adding an eluent to the sample pad, so that the amplicons of the target nucleic acid in the sample pad flow to the starting pad of the detection body along with the eluent, and then flow to the at least one control line and the at least one test line of the detection body from the starting pad, wherein the amplicons of the target nucleic acid is captured on the at least one test line.

**8.** The nucleic acid detection method as claimed in claim 7, wherein after completing the sample adding step, further comprises a first cleaning step, wherein the first cleaning step is to add a first cleaning liquid to the sample pad after the liquid absorption area of the motion body corresponds to the sample pad.

**9.** The nucleic acid detection method as claimed in claim 7, wherein after completing the nucleic acid amplification step, further comprises a second cleaning step, wherein the second cleaning step is to add a second cleaning liquid to the sample pad after of the liquid absorption area of the motion body corresponds to the sample pad.

**10.** The nucleic acid detection method as claimed in claim 7, wherein after completing the sample adding step, further comprises a first cleaning step, and after completing the nucleic acid amplification step, further comprises a second cleaning step; wherein the first cleaning step is to add a first cleaning liquid to the sample pad after the liquid absorption area of the motion body corresponds to the sample pad; wherein the second cleaning step is to add a second cleaning liquid to the sample pad after the liquid absorption area of the motion body corresponds to the sample pad.
